# EUROPEAN PATENT APPLICATION

(11) **EP 4 218 534 A1**
(43) Date of publication of application: **02.08.2023**
(21) Application number: 22153659.2
(22) Date of filing: 27.01.2022
(51) Int. Cl.: A61B 1/015, A61B 1/12, A61B 1/267, A61B 1/00

(54) **SUCTION-FLUSHING-SYSTEM AND MEDICAL INSTRUMENT**

(71) Applicant: Medizinische Hochschule Hannover, 30625 Hannover (DE)
(72) Inventor: Raymondos, Prof. Dr. Konstantinos, 30655 Hannover (DE)
(74) Representative: Kröncke, Rolf

(57) **Abstract**

The invention relates to a suction-flushing-system for a medical instrument having at least one optical device in a distal region adapted for insertion into a patient, which is cleanable by means of the suction-flushing-system. The invention also relates to a medical instrument having such a suction-flushing-system.

## Description

The invention relates to a suction-flushing-system for a medical instrument having at least one optical device in a distal region adapted for insertion into a patient, which is cleanable by means of the suction-flushing-system. The invention also relates to a medical instrument having such a suction-flushing-system.

A medical instrument is known from EP 2 720 745 B1. The medical instrument may in particular be an instrument of endoscopy (endoscope), a video tubus or a video laryngoscope. Endoscopy makes it possible to examine body cavities or hollow organs in the patient's body by inserting a tube- or pipe-guided (video) optical system. This also includes the examination of the larynx and endotracheal intubation using (video) laryngoscopy. In these examinations, the contamination of the optics by body secretions, coagulated blood residues as well as solid components, e.g. food residues, can be problematic, because it limits the view of the tissues to be examined accordingly.

It is an object of the invention to provide an improved suction-flushing system for a medical instrument, which ensures efficient cleaning of an optical device of the medical instrument with the simplest possible operation.

This object is achieved by a suction-flushing-system for a medical instrument having, in a distal region adapted for insertion into a patient, at least one optical device which is cleanable by means of the suction-flushing-system, the suction-flushing-system having at least one flushing medium outlet opening for discharging a flushing medium jet onto the outer surface of the optical device and at least one suction opening for withdrawing by suction at least liquid components from the outer surface of the optical device. The system according to the invention allows a precisely targeted and thus very efficient cleaning of the optical device, hereinafter also referred to as optics, by targeted ejection of the flushing medium jet onto the outer surface of the optical device and also by suction of the liquid components from the outer surface of the optical device. Thereby, at least a part of the emitted flushing medium can be removed again immediately through the suction opening, so that a flushing-suction cycle can be created. The flushing medium can be, for example, a flushing liquid.

The medical instrument may be, for example, a video laryngoscope, a videotube, a flexible endoscope or a rigid endoscope. Such a medical instrument may have as an optical device, for example, a video camera that outputs captured video images wired or wirelessly to a display device. The optical device may also be a lens and/or a light guide. The optical device is located in the distal region of the medical instrument, for example, at the free end of the distal region or slightly recessed.

The flushing medium outlet opening can be connected to a flushing medium supply tank or a flushing medium supply opening of the suction-flushing-system via a flushing medium channel, e.g. a tube or another type of hollow channel. The flushing medium supply tank or flushing medium supply opening may be located at a region remote from the distal region of the medical instrument, for example, at the proximal region, such as at or near a handle of the medical instrument. The suction opening may be connected to a vacuum port of the suction-flushing-system via a suction channel, such as a tube or other type of hollow channel. The vacuum port may be located at a portion remote from the distal region of the medical instrument, for example in the proximal region, such as at or near a handle of the medical instrument. The distal region is considered to be those parts that are located at the distant (distal) end as seen by the user of the medical instrument. Accordingly, the proximal area is the area facing the user (close to the user). The suction channel may be structurally integrated within the flushing medium channel, for example in the form of a pipe-in-pipe-design. It is also possible that the flushing medium channel is structurally integrated within the suction channel.

In addition, in contrast to known flushing or suctioning mechanisms, where only one channel or opening is present, the present invention provides significant advantages by providing a suctioning mechanism which is independent from the flushing mechanism, which means that the flushing medium can be provided through a separate channel which is separate from a suction channel.

According to an advantageous embodiment of the invention, the suction-flushing-system is arranged for simultaneously performing the ejection of the flushing medium jet onto the outer surface of the optical device via the flushing medium outlet opening and for withdrawing by suction at least liquid components from the optical device through the suction opening. In this way, all or at least most of the flushing medium can be sucked out again directly. In this way, additional irritation of the patient can be avoided.

According to an advantageous embodiment of the invention, the suction-flushing-system is arranged to suck back the predominant share of the flushing medium, which has been ejected via the flushing medium outlet opening, through the suction opening. For example, more than 50% of the flushing medium ejected through the flushing medium outlet opening can be immediately sucked away again through the suction opening, or at least 75% or at least 90%.

According to an advantageous embodiment of the invention, the suction-flushing-system is arranged for one-hand operation, in particular for one-finger operation, at least for activating the ejection of flushing medium through the flushing medium outlet opening and for activating the suction of at least liquid components through the suction opening, in particular for one-hand operation or one-finger operation by the hand controlling the medical instrument. This has the advantage that no additional person is required to operate the suction-flushing-system. In addition, the second hand of the user can be used for other activities such as performing intubation or other interventions.

According to an advantageous embodiment of the invention, the flushing medium outlet opening has a smaller cross section, in particular in the form of a nozzle, than a flushing channel providing the flushing medium to the flushing medium outlet opening. In this way, the flushing medium jet can be emitted through the flushing medium outlet opening at high pressure, similar to a high-pressure cleaner. The reduction in cross-section increases the resistance of the flushing channel, which also has the advantage that the amount of liquid required for an effective high-pressure flushing jet can be kept low. Therefore, the suction-flushing-system according to the invention requires only a relatively small supply tank for flushing medium.

According to an advantageous embodiment of the invention, the suction-flushing-system has at least one mechanical coupling element which is arranged for form fit mechanical coupling of the suction-flushing-system to the medical instrument. This allows for easy, reliable and intuitive attachment of the suction-flushing-system to the medical instrument. At least one mechanical receiving element can be present on the medical instrument, which is formed as a counterpart to the mechanical coupling element and is arranged for form fit mechanical coupling with the mechanical coupling element.

Accordingly, the suction-flushing-system can be arranged as a retrofittable assembly that can be attached to the medical instrument by the user. The suction-flushing-system can be arranged, for example, as a disposable one-way item that is disposed of after a single use.

In a further embodiment, the suction-flushing-system can also be a permanent component of the medical instrument, e.g. by being structurally integrated into the medical instrument or at least permanently attached thereto.

According to an advantageous embodiment of the invention, the flushing medium outlet opening has a flushing medium ejection direction which is aligned with the outer surface of the optical device. This allows a particularly efficient cleaning of the optical device by direct blasting of the flushing medium jet onto the outer surface of the optical device.

According to an advantageous embodiment of the invention, the flushing medium outlet opening is arranged immediately adjacent to the outer surface of the optical device. In this way, the flushing medium jet has nearly no pressure loss compared to the initial pressure when exiting the flushing medium outlet opening until it hits the optical device.

According to an advantageous embodiment of the invention, the suction opening is arranged immediately adjacent to the outer surface of the optical device. This ensures efficient re-suction of the flushing medium through the suction opening.

According to an advantageous embodiment of the invention, the suction-flushing-system has a storage tank for storing flushing medium. This has the advantage that no additional hose or other supply line has to be connected to the medical instrument or the suction-flushing system, which simplifies handling for the user. Due to the very efficient cleaning effect of the flushing medium jet and the low liquid consumption, the storage tank can be relatively small, e.g. with a storage volume of a maximum of 25 ml or a maximum of 10 ml or a maximum of 5 ml or a maximum of 3 ml or a maximum of 1 ml.

According to an advantageous embodiment of the invention, the suction-flushing-system has a first manual actuating element, by the manual actuation of which the ejection of flushing medium from the flushing medium outlet opening can be activated. The first manual actuating element can, for example, be in the form of an actuating button which can be actuated by pressing with a finger.

According to an advantageous embodiment of the invention, the first manual actuating element is arranged for manual pressurization of the storage tank. This has the advantage that the pressure required for the flushing medium jet can be generated directly by the manual pressurization. The suction-flushing-system can therefore be realized without an externally powered pump, which supports a simple and compact design of the suction-flushing-system. The manual actuating element can be arranged, for example, for direct pressurization of the supply tank, or for indirect pressurization via a mechanical connection element that couples the manual actuating element to the supply tank.

According to an advantageous embodiment of the invention, the suction-flushing-system has a second manual actuating element, by the manual actuation of which the suction of at least liquid components through the suction opening can be activated. The second manual actuating element can, for example, be arranged similarly to the first manual actuating element, e.g. as an actuating button to be pressed by the user. In a particularly advantageous embodiment of the invention, the second manual actuating element can be arranged as a pneumatic opening which is in communicating connection with a vacuum port of the suction-flushing-system. Through the pneumatic opening, a defined leakage is generated in the non-actuated case, through which air is sucked in from the environment. For manual actuation of such a second manual actuating element, the user can cover the pneumatic opening with a finger and thus at least partially close it. This changes the pressure conditions in the vacuum lines in such a way that the vacuum present at the vacuum port essentially propagates to the suction opening and the desired suction of at least liquid components can accordingly be carried out there. Thus, the flushing medium and other components in the environment can be sucked off through the suction opening.

According to an advantageous embodiment of the invention, the second actuating element is structurally integrated with the first actuating element as a combined actuating element, so that the ejection of flushing medium and/or the suction of at least liquid constituents can be selectively activated by different manual modes of actuation of the combined actuating element. In this way, a particularly simple operation of the flushing and suction functions is provided for the user, which can be performed with one finger. In particular, even when the actuating element is actuated in such a way that flushing medium is dispensed, simultaneous suction through the suction opening is ensured, i.e. the suction process is not interrupted by this.

For example, the combined actuating element allows one of the following operating modes to be selected by different manual actuation modes:
a) no suction of at least liquid constituents and no ejection of flushing medium,
b) suction of at least liquid components without ejection of flushing medium,
c) the suction of at least liquid components with simultaneous ejection of flushing medium.

According to an advantageous embodiment of the invention, the different manual actuation modes can be selected by varying a manual pressurization of the combined actuating element. For example, by not actuating the combined actuating element, the operating mode a) can be selected, by light manual pressurization the operating mode b) can be selected and by stronger manual pressurization the operating mode c) can be selected.

According to an advantageous embodiment of the invention, the suction-flushing-system has a pump for generating a suctioning effect, e.g. a vacuum or underpressure, at the least one suction opening. With such a pump the suction-flushing-system can be operated without the need of connecting the system to an external vacuum source. The vacuum effect can be generated by the own pump of the suction-flushing-system. In such case, the at least one suction opening can be connected via the suction channel to the pump. The pump can be an electrically driven pump.

It is also possible that the pump is a manually driven (operated) pump, which does not need electric energy supply. The pump can be structurally integrated in the suction-flushing-system or in a part of the medical instrument which uses the suction-flushing-system.

In case of a manually driven pump an advantageous embodiment for such a pump can comprise a compressible chamber which can be compressed by manual pressure. The chamber has an automatic decompression functionality which enables the chamber, when it is compressed by manual pressure, to automatically restore into the uncompressed shape. During such automatic recovery of the uncompressed shape, the chamber generates the desired vacuum for withdrawing by suction at least liquid components through the at least one suction opening. The automatic decompression functionality can be realized, for example, by a compressible spring and/or by a design of the chamber itself that it has a recovering spring effect.

According to an advantageous embodiment of the invention, the pump is connectable to the at least one suction opening and an additional emission channel through a valve mechanism, which selectively connects the pump either with the at least one suction opening or the additional emission channel depending on the operation mode of the pump. The manually driven pump may be connected to the additional emission channel through the valve mechanism for ejecting the substances contained within the compressible chamber into the separate emission channel which leads these substances out of the patient on which the suction-flushing-system is used. This can be done in an ejection mode of the pump, e.g. during compressing of the chamber. In an evacuation mode, which means when the chamber automatically recovers its decompressed shape, the valve mechanism switches into another state where the at least one suction opening is connected to the compressible chamber.

The aforementioned object is also achieved by a medical instrument with a suction-flushing-system of the type explained above, wherein the medical instrument has at least one optical device in a distal region arranged for insertion into a patient, which optical device can be cleaned by means of the suction-flushing-system. The advantages explained above can also be realized by this.

According to an advantageous embodiment of the invention, the suction-flushing-system has at least one mechanical coupling element and the medical instrument has at least one mechanical receiving element, which is formed as a counterpart to the mechanical coupling element and is arranged for form fit mechanical coupling of the suction-flushing-system to the medical instrument. The defined form fit mechanical coupling ensures that the flushing medium outlet opening is correctly placed and aligned with respect to the optical device, so that it is ensured that the flushing medium jet directly impinges on the outer surface of the optical device. The same applies to the placement and alignment of the suction opening, which is also always correctly aligned due to the form fit mechanical coupling.

The invention is suitable, for example, for the following areas of application.

### Endoscopy

When the invention is used in endoscopy in the lungs or gastrointestinal tract, the jet of flushing medium is aimed directly at the optics, so that mechanical cleaning can be sufficient just by the jet of flushing medium. An additional cleaning effect is achieved by aspirating at least liquid components through the suction opening.

Flushing and suction can thus take place simultaneously, since there are separate openings, namely the flushing medium outlet opening and the suction opening. The flushing liquid can therefore be removed again directly, which can prevent irritation with subsequent obstruction of the airways by the non-aspirated flushing liquid. The suction-flushing-system can be operated with the hand that controls the endoscope.

### Video tubes

When the invention is used in video tubes, flushing and suctioning can be performed specifically and effectively at the optic. There is no need to use a separate suction catheter, therefore ventilation through this lumen does not have to be interrupted. Thus, the risk of collapsed lung areas and atelectasis can be significantly reduced.

Simultaneous flushing and targeted suctioning at the optic is possible in a simple way. Therefore, even with videotubes, the flushing fluid can be removed directly, which avoids irritation with subsequent obstruction of the airways.

### Videolaryngoscopy

If the optic view is blocked, videolaryngoscopic intubation is impossible. The video function is canceled and only conventional, direct laryngoscopy can then be performed without vision via the optic. However, this is only possible if the curvature of the video laryngoscope blade is Macintosh-like. This Macintosh-like curvature can then allow a classic direct view of the glottis and then intubation without optic vision. All of this can be avoided when using the invention in videolaryngoscopy.

The invention thus solves various problems in five technical areas, which prevent effective and safe cleaning of the optics during endoscopy, video tubes and especially video laryngoscopy in conventional medical instruments:
1. Suction and flushing are not aligned with the optics and are therefore not efficient.
2. Suction and simultaneous flushing are not possible, even with assistance, in endoscopy and videolaryngoscopy, and are only possible to a very limited extent in videotubes.
3. The optic cannot be flushed and sucked at the same time with the same hand controlling the medical device, so that the second hand need to be used for this work and is not available for intubation or other intervention.
4. The resistance of the flushing line is not high enough to ensure automatic limitation to the necessary volume of flushing fluid and for generating a high flushing pressure.
5. The alignment of the flushing opening on the optics when attaching a disposable suction-flushing-system to a reusable medical device cannot be easily and, consequently, intuitively done and is therefore incorrect or impossible in stress and emergency situations.

### Solutions to the five problem areas

The suction-flushing-system described here enables safe mechanical cleaning of the optics during endoscopy or videolaryngoscopy. Cleaning is performed by targeted suction and simultaneous flushing with the hand guiding the device and with a very small volume of liquid. The suction flushing system is both intuitively attached and intuitively operated, making it suitable for emergency and stress situations:

### 1. Alignment of suction and flushing to the optics.

The suction opening is directly adjacent to the optics and is aligned with them in such a way that cleaning can be carried out effectively by suction. The flushing medium outlet is aimed precisely at the surface of the optics, so that even a small amount of liquid can be used to rinse the optics free. When flushing and suctioning at the same time, the entire flushing liquid or the major share thereof is directly aspirated again. In this way, for example, additional irritation of the respiratory tract can be avoided.

### 2. Simultaneous suction and flushing

Suction and simultaneous flushing are ensured by separate suction opening and flushing medium outlet opening and the separate routing of the associated lines, e.g. hoses or channels.

### 3. One-handed control

With one finger of the hand controlling the device, suction can be activated and, in addition, simultaneous flushing can be activated easily and intuitively, so that the second hand is available for intubation or other intervention. A mechanism for activating the flushing can be placed at the pneumatic opening to be closed for activating the suction. This can be realized electrically or mechanically, e.g. by opening a flushing line or by pushing out an annular tank. By placing a squeezable tank directly at the pneumatic opening to be closed for suction activation, the flushing can easily and intuitively be performed simultaneously with the suction.

### 4. High flushing line resistance

Due to the high resistance of the flushing line, the flushing liquid volume is automatically reduced and limited to what is absolutely necessary. At the same time, this ensures a high flushing pressure, through which even gel-like, sticky and/or solid substances can be removed mechanically. This can be achieved, for example, by a small diameter of the flushing channel and/or the flushing medium outlet opening close to the optics.

### 5. Correct attachment of the suction-flushing-system

Correct attachment of the suction-flushing-system (e.g. disposable article) to a medical instrument (e.g. reusable device) is also possible intuitively. This ensures the desired alignment of the suction opening and the flushing medium outlet opening on the optic even in stress and emergency situations and thus also guarantees the functionality of the optic during use on the patient. This can be achieved, for example, by matching the parts to be assembled on the optic like a puzzle and a missing piece of the puzzle. Incorrect attachment and thus incorrect alignment of the openings on the optics can be reliably ruled out.

This also applies to suction-flushing-systems integrated into disposable blades.

During videolaryngoscopic intubation, the suction-flushing-system guarantees an unobstructed view of the vocal folds even in the presence of large amounts of fluid in front of the optics, thus creating the prerequisite for successful airway protection by means of endotracheal intubation even in special emergency situations in which fluid accumulates in front of the laryngeal inlet. This can be mucus secretions, bleeding in the upper respiratory tract and/or regurgitated stomach contents or vomit.

In addition, the flushing fluid is also removed immediately so that it cannot block the view or cause irritation with subsequent airway obstruction. This is particularly important for awake intubation or endoscopy of the airway in awake patients.

The invention therefore proposes a combined suction and flushing device that can be activated with the same hand that controls the medical instrument. In this respect, no second hand is required to operate the suction-flushing-system.

To activate the suction and flushing function, a finger-lockable opening is provided on the suction-flushing system or on the medical instrument, which can be surrounded by a ring-shaped tank. Closing the opening creates a vacuum which activates the suction mechanism, and at the same time, stronger pressure from the closing finger can empty the ring-shaped tank, which initiates a flushing process. The flushing channel is arranged in such a way that it directs the flushing jet directly to the optics of the device. Furthermore, the flushing channel can be embedded in the suction channel, so that a parallel suction of the flushing liquid is possible.

The invention is explained in more detail below by means of exemplary embodiments with the use of drawings.

They show
- Figure 1: a suction-flushing-system and a medical instrument in a schematic block diagram,
- Figure 2: a medical instrument in side view,
- Figure 3: a perspective view of the medical instrument shown in Figure 2,
- Figure 4: a detail enlargement of the distal area of the medical instrument according to Figure 2,
- Figure 5: the distal area of the medical instrument according to Figure 2 without suction-flushing-system,
- Figure 6: the distal area of the medical instrument according to Figure 2 with the suction-flushing-system attached.

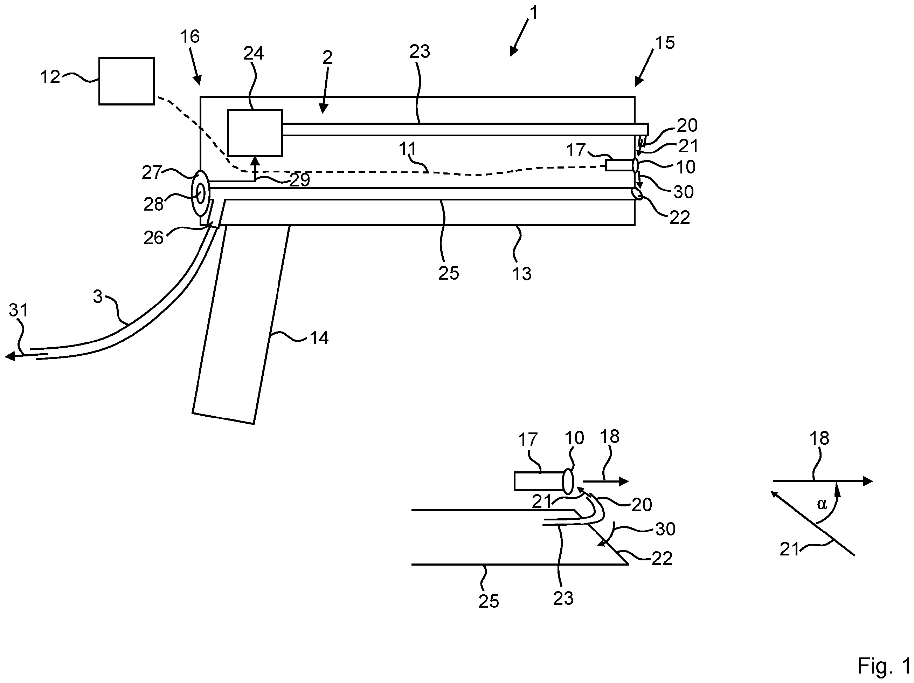

Figure 1 shows a medical instrument 1 having an intervention body 13 and a handling body 14, for example a handle. The intervention body 13 is used to perform medical interventions on a patient, wherein at least a distal, i.e. patient-facing, region 15 is configured for insertion into a patient, e.g. through a natural body opening or, in the context of a surgical procedure, through an artificial opening. The intervention body 13 extends from the distal region 15 to a proximal region 16 facing the user.

The handling body 14 is used for holding and guiding the medical instrument 1 by the user.

The medical instrument has an optical device 17, e.g. a camera sensor, in the distal region 15. The optical device 17 is connected to a display device 12, e.g., a screen, via a line 11, which may be routed inside the intervention body 13 or on the outside thereof. For example, such an optical device 17 can be used in endoscopy to visualize portions of the lungs or gastrointestinal tract on the display device 12. In the case of a videolaryngoscope, the respective airway regions can be visualized depending on the depth of insertion.

The medical instrument 1 is provided with a suction-flushing-system 2. Immediately adjacent to an outer surface 10 of the optical device 17, the suction-flushing system 2 has a flushing medium outlet opening 20, for example in the form of a nozzle. Also immediately adjacent to the outer surface 10 of the optical device 17 is a suction opening 22 for withdrawing by suction at least liquid components from the outer surface 10. For example, the flushing medium outlet opening 20 can emit a flushing medium jet 21 in a jet direction laterally onto the outer surface 10, optionally also with a certain angle in a direction facing away from the patient and onto the outer surface 10. The flushing medium is then immediately eliminated by withdrawing it by suction through the suction opening 22, as indicated by the arrow 30.

The flushing medium outlet opening 20 is connected via a flushing medium channel 23 to a supply tank 24, in which a suitable liquid is stored as flushing medium. The suction opening 22 is connected to a vacuum port 26 via a suction channel 25. A vacuum source can be connected to the vacuum port 26 via a suitable line 3, for example a vacuum source already present in the operating room. In this case, the vacuum port 26 is connected at a point as far away as possible from the suction opening 22 to a combined actuating element 27 which is set up for manual actuation of both the flushing medium ejection and the suction through the suction opening. The actuating element 27 may have a pneumatic opening 28 through which, at least when the pneumatic opening 28 is not covered by the user with a finger, the vacuum supplied via the line 3 is neutralized without creating a relevant suction effect at the suction opening 22. If the pneumatic opening 28 is covered, the vacuum supplied via the line 3 causes media to be sucked in through the suction opening 22 via the suction channel 25.

The combined actuating element 27 is mechanically coupled to the supply tank 24. When greater pressure is applied to the combined actuating element 27, this mechanically pressurizes the supply tank 24, thereby delivering the flushing medium jet 21 through the flushing medium outlet opening 20.

In an advantageous embodiment, which is shown at the bottom right in Figure 1, the flushing channel 23 can be arranged as a line structurally integrated into the suction channel 25. In this way, in particular, a defined direct arrangement of both the flushing medium outlet opening 20 and the suction opening 22 adjacent to the optical device 10 can be realized. The suction opening 22 can be arranged, for example, as a beveled open end of a suction channel 25.

As also shown, the optical device 17 has a viewing direction 18. The flushing medium jet 21 leaves the flushing medium outlet opening 20 in a direction 21. The angle α between the viewing direction 18 and the direction 21 of the flushing medium jet 21 can be in the range of 90 to 180 degrees.

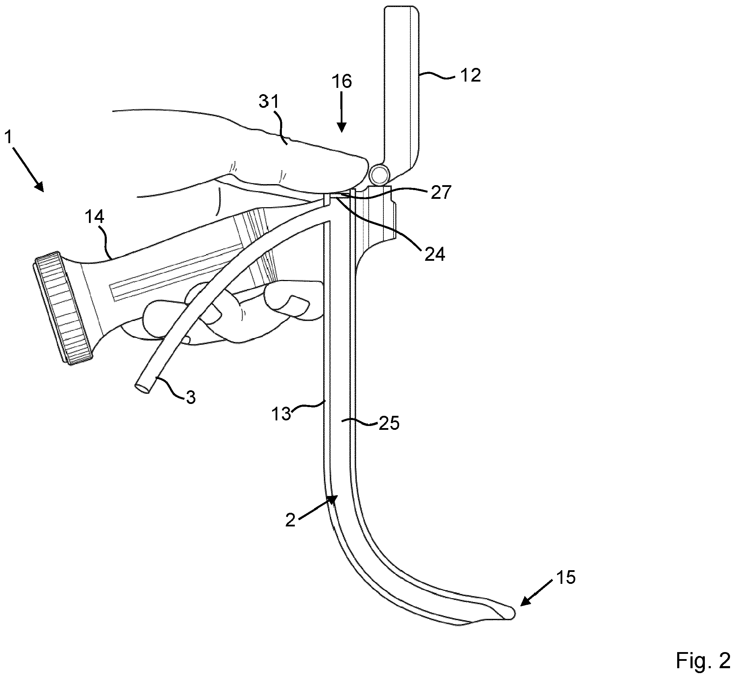

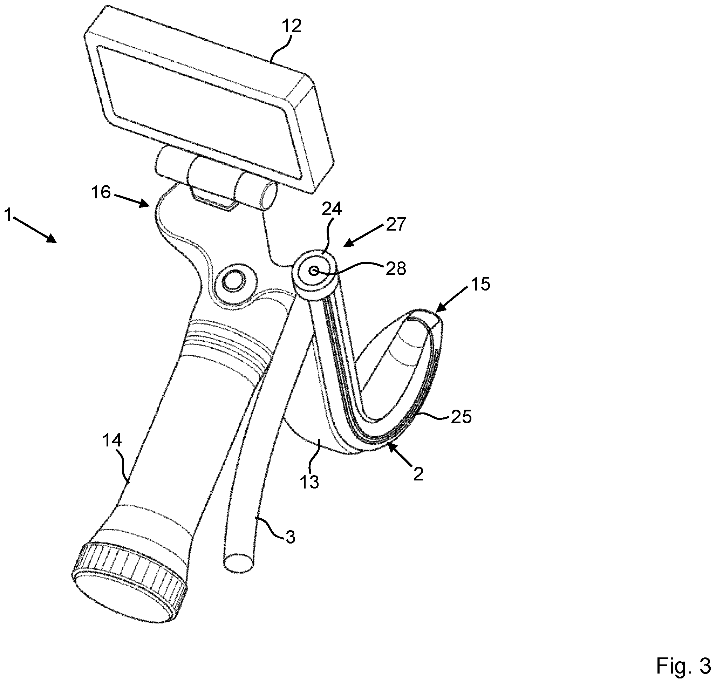

With reference to Figures 2 to 6, advantageous constructive realization possibilities of the medical instrument 1 as well as of the suction-flushing-system 2 and their attachment to the medical instrument 1 are explained. As shown in Figures 2 and 3, the medical instrument 1 can be arranged as a video laryngoscope with a display device 12 attached thereto. In the case of a video laryngoscope, the intervention body 13 is formed as a guide rail for guiding a ventilation tube. It can be seen that the supply tank 24 can advantageously be formed as a ring tank, which is arranged directly on the combined actuating element 27 or forms a part of this actuating element 27. The suction channel 25 extends laterally along the guide rail 13. The flushing channel 23 can also extend laterally along the guide rail 13 or, as already indicated in Figure 1, be structurally integrated into the suction channel 25.

Using the combined actuating element 27, the user can easily choose one of the following operating modes by different manual actuation modes of the combined actuating element 27 by one finger 31:
a) no suction of at least liquid constituents and no ejection of flushing medium: In this case, the user does not put his finger 31 on the actuating element 27, but leaves at least some space between the finger 31 and the actuating element 27. This has the effect that the pneumatic opening 28 is not covered by the finger 31. Also, the supply tank 24 is not pressurized by the finger 31.
b) suction of at least liquid components without ejection of flushing medium: In this case, the finger 31 is placed on the actuating element 27 with only little pressure which is sufficient to close the pneumatic opening 28. However, the pressure of the finger 31 is not sufficient for compressing the supply tank 24.
c) the suction of at least liquid components with simultaneous ejection of flushing medium:
   In this case, the pressure of the finger 31 on the actuating element 27 is significantly increased. Then the pneumatic opening 28 is still closed by the finger 31. In addition, the supply tank 24 is pressurized in a way that the flushing medium is pressed through the flushing medium channel 23 to the flushing medium outlet opening 20. The longer the high pressure on the supply tank 24 is maintained, the more the supply tank 24 is compressed and deformed, until the supply tank 24 is completely scrunched when the whole fluid medium content has been pressed out of the supply tank 24.

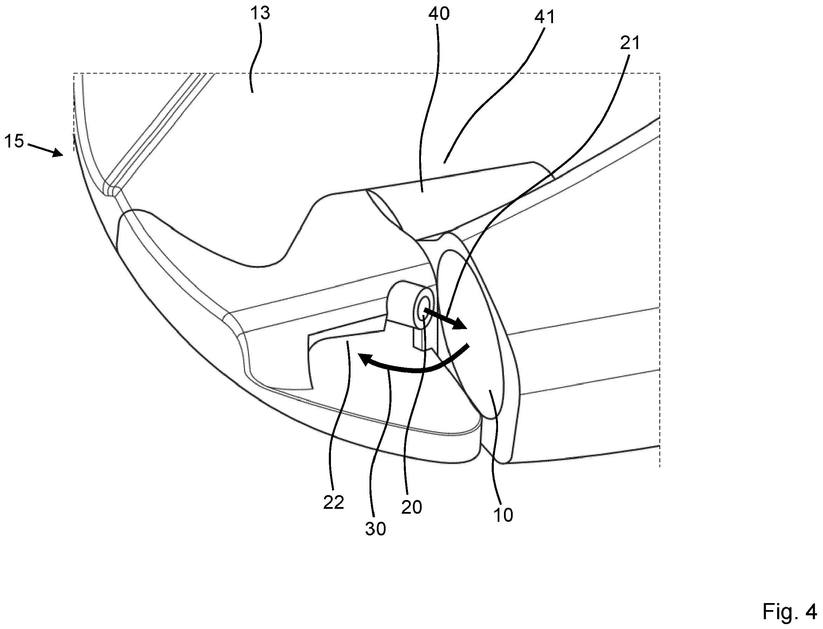

Figure 4 shows an enlarged sectional view of the distal end 15 of the medical instrument 1 or of its intervention body 13. In this area, the intervention body 13 is specifically arranged as a counterpart to a corresponding shape of a distal coupling area 40 of the suction-flushing-system 2. This coupling area 40 has one or more form-locking elements which engage in a form-locking manner in corresponding formings of the area 41 of the intervention body 13.

As can be seen in Figure 4, the flushing medium outlet opening 20 emits the flushing medium jet 21 directly onto the outer surface 10. The flushing medium is then immediately eliminated by withdrawing it by suction through the suction opening 22, as indicated by the arrow 30.

It can also be seen the advantageous structural integration of the distal area of the suction-flushing-system 2 with the flushing medium outlet opening 20 and the suction opening 22 arranged directly next to each other. The flushing medium outlet opening 20 is aligned directly with the outer surface 10 of the optical device 17 to be cleaned.

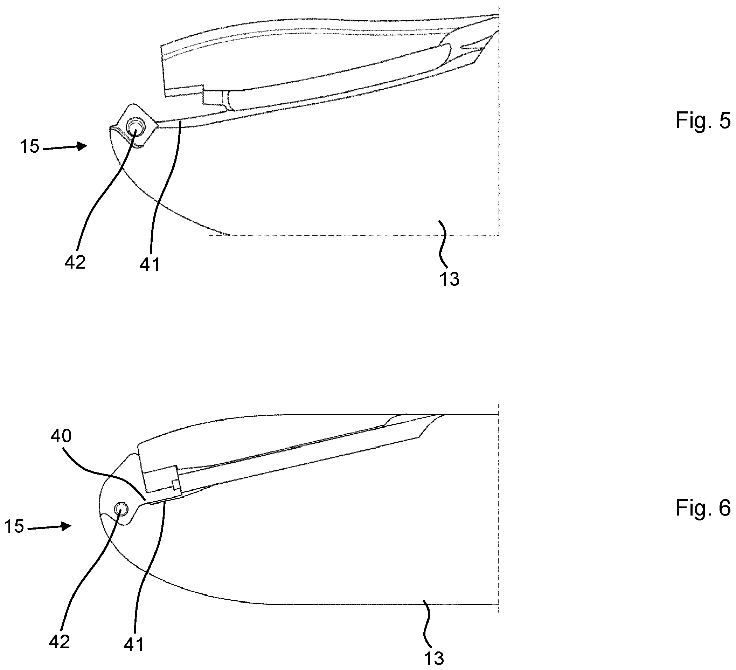

With reference to Figures 5 and 6, a further possibility of advantageously attaching the distal portion of the suction-flushing-system 2 to the intervention body 13 is explained. On the intervention body 13, for example, an additional fixing element 42, e.g. in the form of a latching lug or other nub 42, can be arranged, which engages in a corresponding recess of the distal section 40 of the suction-flushing-system 2 attached thereto and thus positively fixes it there.

## Claims

1. A suction-flushing-system (2) for a medical instrument (1) having, in a distal region (15) adapted for insertion into a patient, at least one optical device (17) which is cleanable by means of the suction-flushing-system (2), the suction-flushing-system (2) having at least one flushing medium outlet opening (20) for discharging a flushing medium jet (21) onto the outer surface (10) of the optical device (17) and at least one suction opening (22) for withdrawing by suction at least liquid components from the outer surface (10) of the optical device (17).

2. Suction-flushing-system according to claim 1, **characterized in that** the suction-flushing-system (2) is arranged for simultaneously performing the ejection of the flushing medium jet (21) onto the outer surface (10) of the optical device (17) via the flushing medium outlet opening (20) and for withdrawing by suction at least liquid components from the optical device (17) through the suction opening (22).

3. A suction-flushing-system according to claim 2, **characterized in that** the suction-flushing-system (2) is arranged to suck back the predominant share of the flushing medium, which has been ejected via the flushing medium outlet opening (20), through the suction opening (22).

4. Suction-flushing-system according to one of the preceding claims, **characterized in that** the suction-flushing-system (2) is arranged for one-hand operation, in particular for one-finger operation, at least for activating the ejection of flushing medium through the flushing medium outlet opening (20) and for activating the suction of at least liquid components through the suction opening (22), in particular for one-hand operation or one-finger operation by the hand controlling the medical instrument (1).

5. Suction-flushing-system according to one of the preceding claims, **characterized in that** the flushing medium outlet opening (20) has a smaller cross section, in particular in the form of a nozzle, than a flushing channel (23) providing the flushing medium to the flushing medium outlet opening (20).

6. Suction-flushing-system according to one of the preceding claims, **characterized in that** the suction-flushing-system (2) has at least one mechanical coupling element (40) which is arranged for form fit mechanical coupling of the suction-flushing-system (2) to the medical instrument (1).

7. Suction-flushing-system according to one of the preceding claims, **characterized in that** the flushing medium outlet opening (20) has a flushing medium ejection direction (21) which is aligned with the outer surface (10) of the optical device (17).

8. Suction-flushing-system according to one of the preceding claims, **characterized in that** the flushing medium outlet opening (20) is arranged immediately adjacent to the outer surface (10) of the optical device (17).

9. Suction-flushing-system according to one of the preceding claims, **characterized in that** the suction opening (22) is arranged immediately adjacent to the outer surface (10) of the optical device (17).

10. Suction-flushing-system according to one of the preceding claims, **characterized in that** the suction-flushing-system (2) has a storage tank (24) for storing flushing medium.

11. Suction-flushing-system according to one of the preceding claims, **characterized in that** the suction-flushing-system (2) has a first manual actuating element (27), by the manual actuation of which the ejection of flushing medium from the flushing medium outlet opening (20) can be activated.

12. Suction-flushing-system according to claim 11, **characterized in that** the first manual actuating element (27) is arranged for manual pressurization of the storage tank (24).

13. Suction-flushing-system according to one of the preceding claims, **characterized in that** the suction-flushing-system (2) has a second manual actuating element (28), by the manual actuation of which the suction of at least liquid components through the suction opening (22) can be activated.

14. Suction-flushing-system according to claim 13, **characterized in that** the second actuating element (28) is structurally integrated with the first actuating element (27) as a combined actuating element (27, 28), so that the ejection of flushing medium and/or the suction of at least liquid constituents can be selectively activated by different manual modes of actuation of the combined actuating element (27, 28).

15. Suction-flushing-system according to claim 14, **characterized in that** by different manual modes of actuation of the combined actuating element (27, 28) one of the following modes of operation can be selected:
a) no suction of at least liquid constituents and no ejection of flushing medium,
b) suction of at least liquid components without ejection of flushing medium,
c) the suction of at least liquid components with simultaneous ejection of flushing medium.

16. Suction-flushing-system according to claim 13 or 14, **characterized in that** the different manual actuation modes are selectable by varying a manual pressurization of the combined actuating element (27, 28).

17. Suction-flushing-system according to one of the preceding claims, **characterized in that** the suction-flushing-system has a pump for generating a suctioning effect at the least one suction opening (22).

18. Suction-flushing-system according to claim 17, **characterized in that** the pump is connectable to the at least one suction opening (22) and an additional emission channel through a valve mechanism, which selectively connects the pump either with the at least one suction opening (22) or the additional emission channel depending on the operation mode of the pump.

19. Medical instrument (1) with a suction-flushing-system (2) according to one of the preceding claims, wherein the medical instrument (1) has at least one optical device (17) in a distal region (15) arranged for insertion into a patient, which optical device (17) is cleanable by means of the suction-flushing-system (2).

20. Medical instrument according to claim 19, **characterized in that** the suction-flushing-system (2) has at least one mechanical coupling element (40) and the medical instrument (1) has at least one mechanical receiving element (41, 42), which is formed as a counterpart to the mechanical coupling element (40) and is arranged for form fit mechanical coupling of the suction-flushing-system (2) to the medical instrument (1).
